# EUROPEAN PATENT APPLICATION

(11) **EP 2 182 356 A1**
(43) Date of publication of application: **05.05.2010**
(21) Application number: 08168250.2
(22) Date of filing: 04.11.2008
(51) Int. Cl.: G01N 33/50

(54) **Use of serum samples for diagnosing prostate cancer by measuring monocyte migration**

(71) Applicant: Koninklijke Philips Electronics N.V., 5621 BA Eindhoven (NL)
(72) Inventor: The designation of the inventor has not yet been filed
(74) Representative: Volmer, Georg

(57) **Abstract**

The present invention relates to the use of samples drawn from patients and their impact on the chemotactic response of monocytes for diagnosing prostate cancer.

## Description

### FIELD OF THE INVENTION

The present invention pertains to the field of diagnosing prostate cancer.

### BACKGROUND OF THE INVENTION

Prostate cancer is the most common male malignancy and the second leading cause of male cancer-related death in the United States. It accounts for 29% of all male cancers and 11 % of all male cancer-related death. Small prostate carcinomas were detected in 30% of men aged 30 to 49 years, in 40% of males over age 50 and in 64% of males aged 60 to 70 years (Sica et al.(1993) J.Urol., 115: 379-385).

In view of the incidence of prostate cancer, a reliable diagnostic method that is capable of predicting this disease at an early stage with high reliability is obviously of major importance.

Currently, prostate cancer screening is typically based on a combination of three diagnostic approaches, namely physical examination, biochemical examination and image analysis.

Physical examination is typically performed by digital rectal examination (DRE). Other approaches include examination of the abdomen by inspection, percussion and palpation in order to detect any distension, palpable renal masses and enlargement of the bladder or tenderness.

Biochemical analysis, to a larger extent, relies on monitoring serum for the level of prostate-specific antigen (PSA). The normally accepted upper level for serum PSA that is determined by monoclonal antibody in an immuno assay is 4 ng/ml. However, some men with prostate cancer may have normal levels of PSA and elevated levels of PSA may also be due to other factors, such as age, infarction etc. Thus, PSA monitoring as well as analysis of other biochemical factors is in itself insufficient to either exclude or confirm the presence of a tumor. Moreover, PSA is without use with respect to differentiating aggressive and non-aggressive forms of the cancer.

As a consequence, the two aforementioned diagnostic approaches, namely DRE and PSA monitoring are usually supported by trans-rectal ultrasound (TRUS) guided biopsy analysis. The biopsy is targeted to a focal area of abnormal echogenicity or to area of palpable abnormality. As the area of tumor development may not easily be identifiable in the trans-rectal ultrasound analysis, one will take 6, 12 or sometimes even more biopsies from different areas of the prostate during a normal ultrasound scan. These targeted biopsies are then followed by subsequent histological analysis. Even though histological analysis, if performed by an experienced histopathologist, may usually give a good indication of whether a tumor is malignant or benign, the number of false-negative diagnoses is considerable. Thus, each biopsy may miss the presence of prostate cancer including even the most aggressive forms. TRUS-guided needle biopsy can miss up to 34% of clinically localized prostate cancers and about 10 to 19% of patients with initially negative needle biopsy were diagnosed with prostate on a second biopsy (Kietch et al (1994), J. Urol., 151: 1571-1574).

Another issue with TRUS-guided biopsy and the subsequent histopathological analysis is that it is rather burdensome for patients suspected of suffering from prostate cancer. This is a particularly important aspect, as there are a considerable number of benign tumors which do not necessarily require treatment. However, the potential transformation of a tumor from benignto malignant needs to be carefully monitored in order to ensure that treatment is initiated at appropriate time. It is obvious that the painful approach of TRUS-guided biopsy is not the prime choice for monitoring tumor development over time in patients.

As a result, many men suffering from benign prostate hyperplasia are currently "watchfully observed" for symptoms to arise.

In view of this situation there is a continuing need for diagnostic approaches that allow detection of the presence of prostate tumors and grading their stage of development, i.e. benign versus malignant.

The same applies to other cancer types which as prostate cancer may be classified as adenocarcinomas. For such cancers which include breast cancer, lung cancer and colorectal cancer, there is also an interest in approaches that allow detection of the presence of the respective tumor and grading their stage of development.

### OBJECT AND SUMMARY OF THE INVENTION

It is one objective of the present invention to provide a method of diagnosis that allows for diagnosing the presence or likely occurrence of adenocarcinoma-related cancers. It is a further objective of the present invention to provide diagnostic approaches that allow monitoring of the transition of adenocarcinoma-related tumors from benignancy to malignancy. Yet another objective of the present invention is to provide methods of acquiring the data that can be used in the aforementioned described methods of diagnosis.

These and other objectives of the present invention, as they will become apparent from the ensuing description are solved by the subject matter of the independent claims. The dependent claims relate to some of the preferred embodiments of the present invention.

The present invention is based on the finding that a migratory response of monocytes towards serum samples obtained from patients suffering from adenocarcinoma-related cancers can be used to diagnose the presence and/or the state of such a tumor. The present invention therefore in one embodiment relates to a method of diagnosing and/or monitoring development of an adenocarcinoma-related cancer comprising at least the steps of:
a. Providing at least one sample of at least one individual suspected of suffering from or of developing an adenocarcinoma-related cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from at least one healthy individual;
e. Comparing the chemotactic responses of said blood cells;
f. Deciding on the occurrence and/or state of an adenocarcinoma-related cancer on the basis of the results obtained in e.

Another embodiment of the present invention relates to a method of diagnosing and/or monitoring development of an adenocarcinoma-related cancer comprising at least the steps of:
a. Providing at least one sample of at least one individual suspected of suffering from or of developing an adenocarcinoma-related cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from the same at least one individual at later point in time;
e. Comparing the chemotactic responses of said blood cells;
f. Deciding on the state of an adenocarcinoma-related cancer on the basis of the results obtained in e.

The aforementioned methods are preferably conducted on samples that are not in direct contact with the human body.

In a preferred embodiment, the results obtained in step e. are used for deciding on the benignancy or malignancy of an adenocarcinoma-related tumor.

Adenocarcinoma-related cancers typically will comprise breast cancer, colon cancer, prostate cancer, lung cancer, kidney cancer, stomach cancers, pancreas cancer, cervix cancer, vaginal cancer, some head and neck cancers and urachus cancer.

The methods described hereinafter are in one preferred embodiment conducted on breast cancer, colon and rectal cancer or prostate cancer.

Yet another embodiment of the present invention relates to a method of data acquisition comprising at least the steps of:
a. Providing at least a sample of at least one individual suspected of suffering from or of developing an adenocarcinoma-related cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from at least one healthy individual;
e. Comparing the chemotactic responses of said blood cells.

A further embodiment of the present invention relates to a method of data acquisition comprising at least the steps of:
a. Providing at least a sample of at least one individual suspected of suffering from or of developing an adenocarcinoma-related cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from the same at least one individual at later point in time;
f. Comparing the chemotactic responses of said blood cells.

In the aforementioned methods of data acquisition, the sample in one embodiment preferably is not in direct contact with the human body.

The methods described hereinafter are in one preferred embodiment conducted on breast cancer, colorectal cancer or prostate cancer.

In one of the preferred embodiments of the present invention, the aforementioned samples that are to be used in the methods of diagnosis and methods of data acquisition are human blood samples. Preferably, the samples are serum samples.

The blood cells to be used in the afore described methods of diagnosis and method of data acquisition are chemotaxing blood cells including monocytes, T-lymphocytes, neutrophils, stem cells and progenitor cells. One can also use chemotaxing cultured cell lines of monocytes, granulocytes,lymphocytes, as well as myeloid and lymphoid precursors. These blood cells are preferably not derived from the patients one is going to analyze in the methods in accordance with the invention.

Preferably one uses monocytes. Typically they are preferably selected from monocytic cell culture lines. Such cell lines comprise U937 monocytes and THP-1 monocytes. Other monocytic cell lines can be found in Human Cell Culture, (2002), Vol. 3, pages 237-257, Springer Netherlands (ISNN 1389-2142). In one of the preferred embodiments of the present invention, the monocytes used are not tumor-associated macrophages.

The sample as described above may act on the monocytes as described above as a chemotactic agent, i.e. either a chemoattractant or a chemorepellant.

In a preferred embodiment, monitoring the chemotactic response of monocytes to sample thus relates to determining of whether monocytes migrate towards or away from the sample.

### BRIEF DESCRIPTION OF THE DRAWINGS

Figure 1 depicts a device that may be used to determine the migratory response of e.g. monocytes towards a sample obtained from patients suspected of suffering from prostate cancer.
Figure 2 depicts the device of Figure 1 at different settings.
Figure 3 depicts another device that may be used to monitor the chemotactic response of e.g. monocytes towards serum samples obtained from patients suspected of suffering from prostate cancer.
Figures 4 to 7 are embodiments of the device depicted in Figure 3.
Figure 8(A) depicts a Boyden Chamber. Monocytes are placed in the upper chamber while patient serum is placed in the lower chamber.
Figure 8(B) depicts typical results of a Boyden assay utilizing cell culture monocyte in serum from healthy individuals. The panel shows the bottom of the filter, post-assay, after washing/removal of unbound cell and subsequent cell staining with Wright's stain. Panel 1 contains no serum and hence minimal monocyte migration. Stain cells are indicated with red arrows to add visualization. Panels 2 to 4 contain increasing concentrations of serum diluted in cell medium 1 to 15 (panel 2), 1 to 3 (panel 3) and 2 to 3 (panel 4).
Figure 9 depicts theoretical results of a migration assay utilizing patient serum from a healthy individual (left bar), patient with a primary, non-aggressive tumor (middle bar) and a patient with a tumor that has progressed into angiogenesis and invades surrounding tissue (right bar).
Figure 10 (A) depicts the migration of THP-1 monocytes which have been conditioned in different media mimicking the sera composition of patients suffering from colorectal tumors of different stages.
Figure 10 (B) depicts the migration of THP-1 monocytes which have been conditioned in different media mimicking the sera composition of patients suffering from prostate tumors of different stages.

### DETAILED DESCRIPTION OF EMBODIMENTS

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings as described are only schematic and nonlimiting. In the drawings, the size of some of the elements may be exaggerated and not drawn on scale for illustrative purposes.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±10%, and preferably of ±5%.

Further definitions of terms will be given in the following in the context of which the terms are used.

Tumors of the prostate have been described in the past to interact differently with cells of the immune system depending on the benignancy or malignancy of the tumor, i.e. the state of development of the tumor. The same applied to other adenocarcinoma-related tumors such as colon and rectal cancer, lung cancer and breast cancer. Thus, during early development of the tumor, when the tumor may still be considered as benign, monocytes, the physiological function of which are usually to fight inflammatory attacks, are repelled from such tumors.

As tumor development continues, the tumor needs to recruit nutritional support and to invade into other tissues. Blood vessel formation of tumors is typically described as angiogenesis and the event when angiogenesis of a tumor starts is typically referred to as the "angiogenic switch" being a hallmark of progression of the tumor from a benign to a malignant state. Such malignant tumors are often characterized in that they recruit monocytes, in order to e.g. degrade and remodel extra-cellular matrix structures allowing spreading of the tumor. Tumors and the monocytes contained within also secrete various growth factors and cytokines that facilitate the formation and growth of novel vasculature.

The present invention is based on the finding that the different states of an adenocarcinoma-related tumor are reflected by different factors being present in the blood of a patient suffering from such tumors and that this differential blood composition can be used to monitor the migratory behavior of e.g. monocytes. Solely for the purpose of an example, a benign prostate cancer tumor may induce a secretion of factors into the bloodstream which act as inhibitors of chemotaxis and thus would prevent e.g. monocytes from migrating towards the corresponding serum samples. On the other side, a prostate tumor, which has progressed beyond the angiogenic switch, may be linked to growth factors and cell adhesion factors such as VEGF or VCAM. Blood samples of such patients suffering from malignant tumors may thus comprise chemoattractant molecules and could thus be used to induce a migration of e.g. monocytes towards such sample.

By comparing the migratory behavior of blood cells such as monocytes towards blood and preferably serum samples obtained from e.g. patients suspected of suffering from prostate cancer and healthy control individuals, one could thus decide on the presence of a prostate cancer tumor as well as of its stage, i.e. stage of development. By repeatedly drawing blood samples from the same individual patient over time, one could further monitor the progression from benignancy to malignancy by determining a change in the chemotactic behavior of e.g. monocytes towards the respective blood samples.

As mentioned above, the present invention in one embodiment thus relates to a method of diagnosing and/or monitoring development of an adenocarcinoma-related cancer comprising at least the steps of:
a. Providing at least a sample of at least one individual suspected of suffering from or of developing an adenocarcinoma-related prostate cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from at least one healthy individual;
e. Comparing the chemotactic responses of said blood cells;
f. Deciding on the occurrence and/or state of an adenocarcinoma-related cancer on the basis of the results obtained in e.

The invention further relates to a method of data acquisition comprising at least the steps of:
a. Providing at least a sample of at least one individual suspected of suffering from or of developing an adenocarcinoma-related cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from at least one healthy individual;
e. Comparing the chemotactic responses of said blood cells.

In one embodiment of the present invention, all of the aforementioned steps may be performed outside the human body.

The term "adenocarcinoma-related cancer" refers to cancer types that initially develop as cancers of glandular tissues. Adenocarcinoma-related cancers typically will comprise breast cancer, colon and rectal cancer, prostate cancer, lung cancer, kidney cancer, stomach cancers, pancreas cancer, cervix cancer, vaginal cancer, some head and neck cancers and urachus cancer. The methods described hereinafter are in one preferred embodiment conducted on breast cancer, colorectal cancer or prostate cancer. Even though the term "adenocarcinoma" is typically used to describe the malignant state of a cancer, the term in the present context is used to describe both tumors of benign and malignant state.

The term "sample" refers to samples obtained from a human being. Typically, the term is used to denote blood samples, serum samples, saliva samples and samples derived from other bodily fluids. Typically, the term "sample" does not refer to tissue samples.

Samples derived from human blood and in particular serum are preferred according to the present invention.

The person skilled in the art is familiar with obtaining such samples. For example, blood may be drawn from a patient or healthy individual intravenously or via finger prick. Such samples may then be put into a tube coated with an anticoagulant agent. The blood may then be briefly centrifuged to separate the blood cells from the serum. If desired, centrifugation can be undertaken through a density gradient being made from materials such as percoll, ficoll and/or sucrose. The serum may then be removed and aliquoted, frozen in liquid nitrogen and stored at 80°C.

The term "blood cells" denotes cells as present in the human blood that are known to be capable of chemotaxis if they are contacted with chemoattractants or chemorepellants. Typically such blood cells are monocytes, T-lymphocytes, stem cells, progenitor cells and neutrophils. One can also use chemotaxing cultured cell lines of myeloid and lymphoid lineage. Preferably these blood cells are not derived from the patients which are analyzed in the methods in accordance with the present invention.

Monocytes are preferred for the purposes of the present invention.

The term "monocytes" refers to circulating monocytic cells as being typically present in human blood as well as monocytic cell culture lines. It is to be understood that the term "monocytes" preferably does not refer to macrophages such as tumor-associated macrophages. Macrophages other than monocytes are not circulating, but rather tissue associated.

It is preferred to use monocyte cell culture lines. Such cell lines comprise U937 monocytes and THP-1 monocytes. Other monocytic cell lines can be found in Human Cell Culture, (2002), Vol. 3, pages 237-257, Springer Netherlands (ISNN 1389-2142). The invention in a preferred embodiment relates to the use of monocytes which preferably are not taken from a patient and/or control subject, the serum sample of which is analyzed. Therefore, the use of the aforementioned commercially available and/or established monocyte cell lines is preferred.

The above-described methods of diagnosing and/or monitoring development of prostate cancer as well as the above-described method of data acquisition are characterized in steps a. to f. as well as steps a. to e., respectively.

Thus, one first obtains a sample as described above, which preferably will be a serum sample. The sample will be drawn from a patient who is suspected to suffer from an adenocarcinoma-related cancer such as prostate cancer.

The term "patient" as used hereinafter thus relates to a human individual for which typical diagnostic approaches indicate the presence of an adenocarcinoma-related cancer such as a prostate or a colon cancer tumor, be it of benign or malignant state. The term "patient" further refers to a human being, which by the common standards of a medical practitioner would be considered to display symptoms being characteristic of ongoing adenocarcinoma-related cancer development or potential adenocarcinoma-related cancer development.

The term "healthy individual" refers to a human subject for whom it has been confirmed by typical diagnostic approaches that it does not suffer from an adenocarcinoma-related cancer of any state, be it benign or malignant.

In the above described methods, one thus provides a sample of at least one individual patient suspected from suffering from or developing an adenocarcinoma-related cancer such as prostate cancer, colon cancer such as colorectal cancer or breast cancer. The sample is brought into contact with monocytes, such that a chemotactic response of said monocyte is inducible by said sample. The monocytes may be of an origin as described above. Preferably, the monocytes are not derived from the individual from whom the sample has been obtained. Even more preferably, the monocytes are taken from an established monocyte cell culture line. Bringing such a sample into contact with monocytes such that a chemotactic response of said monocytes is inducible by said sample means that the sample is positioned in such proximity to the monocytes that, in principle, a chemotactic migration towards the sample or away from the sample would be observed if either a chemoattractant or chemorepellant was known to be in the sample.

The term "chemotactic response" or "chemotactic behavior" refers to the migratory reaction of monocytes upon contacting samples in accordance with the invention. Depending on the direction of movement of monocytes induced by a sample in accordance with the invention, samples may be considered to act as chemoratractants or chemorepellants.

The type of reaction depends on the type of chemotactic agent being predominantly present in such samples. In the context of the present invention, the term "chemotactic agent" denotes chemoattractants as well as chemorepellants. Chemoattractents are understood to stimulate migration of cells and particular monocytes towards these agents and thus samples comprising such agents whilst chemorepellants are understood to stimulate the opposite behavior or to at least not include migration towards the sample.

In the context of the present invention the term "chemoattractant" typically denotes chemical substances as well as peptides and proteins as they are known to induce migration of cells. Such chemoattractants typically include growth factors, hormones, peptides, cytokines, chemokines, cell adhesion molecules (CAMs), integrins, drugs and vitamins.

Chemoattractants such as growth factors, hormones, cytokines, chemokines can be preferred, as they are known to attract and induce a migratory behavior in eukaryotic cells such as human or animal mammalian cells.

Typical chemokines include e.g. human growth factor, human nerve growth factor, platelet derived growth factor, TGFβ, EGF, VEGF, TNF-a,TNF-b, FGFs, TGF-a, EPO, IGF-I, IGF-II, IL-1, IL-2, IL-6, IL-8and CSFs as well as integrins and CAMs including VLA-1 to VLA-6, LPAM-1, LFA-1, CR3, CR4, leukointegrin, collagen, laminin, fibronectin, VCAM-1, MAdCAM-1, E-cadherin, ICAM-1, ICAM-2, ICAM-3, C3b, C4b. Other growth factors include CCL 1-28, CXCL 1-16, XCL1, XC:2, CX3CL1, IL 1-22, INF α, β and y, M-CSF, G-CSF, GM-CSF, MIF, any recognized CD marker of haematopoietic cells (CD 1-339), PDGF, NGF, TPO, GDF-8, GDF-9, bFGF, HGF, FGF, DII4 and extracellular matrix components such as collagen, fibronectin and laminin. Another chemoattractant is MCP-1.

The monitoring of the chemotactic response of monocytes with respect to a sample will typically be undertaken by visually monitoring the migration of monocytes. Typically, one may e.g. count the number of monocytes that move towards or away from the sample.

The same type of analysis as described above for the monocyte movement towards samples obtained from a patient may then be undertaken with samples obtained from healthy individuals. Subsequently, the chemotactic behavior is compared and the differences can then be correlated with the presence and stage of adenocarcinoma-related cancer development as it can be deduced from independent diagnostic approaches. In the case of prostate cancer such independent diagnostic approaches may include the aforementioned types of biochemical, image and histological analysis. Once one has identified the type of behavior that is indicative of the presence and/or stage of the specific adenocarcinoma-related cancer development, one can use the migratory behavior, i.e. the chemotactic response to diagnose the occurrence and/or stage of adenocarcinoma-related cancer development independently of such other methods.

The person skilled in the art is aware that the reliability and validity of such a diagnostic test will depend on the number of healthy individuals that have been analyzed. Thus, the more healthy individuals who have been analyzed as to the chemotactic response of monocytes with respect to samples obtained from such individuals, the more reliable are the conclusions based on a difference in the chemotactic response obtained from individual patients.

As mentioned above, another embodiment of the invention relates to a method of diagnosing and/or monitoring development of an adenocarcinoma-related cancer comprising at least the steps of:
a. Providing at least a sample of at least one individual suspected of suffering from or of developing an adenocarcinoma-related cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from the same said at least one individual at later point in time;
e. Comparing the chemotactic responses of said blood cells;
f. Deciding on the state of said adenocarcinoma-related cancer on the basis of the results obtained in e.

Yet another embodiment of the invention relates to a method of data acquisition comprising at least the steps of:
a. Providing at least a sample of at least one individual suspected of suffering from or of developing an adenocarcinoma-related cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from the same said at least one individual at later point in time;
e. Comparing the chemotactic responses of said blood cells.

As regards these latter methods of diagnosis and data acquisition, the above-mentioned definition as regards "sample", patients", monitoring the chemotactic response, monocytes etc, equally apply. Further, embodiments, which have been described above as being preferred, also are preferred for the two latter mentioned methods of diagnosis and of data aquisition. The differences between these latter methods of diagnosis and data acquisition to the aforementioned methods of diagnosis and data acquisition are in that the chemotactic response of monocytes is not compared for samples obtained from a patient versus a healthy individual, but rather for samples obtained from the same patient with the samples being taken at different time points. The different time points may differ by days, months or years. By monitoring the chemotactic response of e.g. monocytes towards samples obtained from the same patient individuals, it is possible to record a change in the state of e.g. a prostate tumor, e.g. the transition of a benign tumor towards a malignant tumor as it may occur as a consequence of an angiogenic switch. Of course, evaluating and assessing the difference in such chemotactic responses for samples obtained from the same patient presumes that suitable control populations have been evaluated before that allow the difference in the migratory response of the samples obtained from the same patient to correlate with a certain occurrence and stage of e.g. prostate cancer development.

Typically, if in any of the above-described methods a migratory behavior is observed that is indicative of movement of e.g. monocytes away from a sample, or at least not towards a sample, this may be considered as being indicative of a benign tumor, if the response as such is indicative of the presence of a tumor at all. This analysis presumes that in the early stages of an adenocarcinoma-related cancer such as prostate cancer development, the serum of a patient will mainly comprise chemorepellants and/or chemotactic inhibitors that should be present as the e.g. monocytes still try to attack the tumor with the tumor trying to avoid this attack by producing either compounds that inhibit monocyte attraction or even induce monocyte rejection.

The migratory response of blood cells to sample derived from patients being suspected of suffering from an adenocarcinoma-related cancer may also be analysed by incubating such blood cells together with such sample, e.g. monocytes together with serum samples. Subsequently, one can then determine whether the migratory behavior of blood cells towards a known chemoattractant such as VEGF, VCAM- lor MCP-1 is changed depending on the incubation of the blood cells with the sample.

If on the other hand, the chemotactic response reveals an attraction of e.g. monocytes towards the sample obtained from a patient, this may be indicative of a malignant adenocarcinoma-related cancer development. The reason being that the malignant prostate cancer tumors are commonly characterized by an angiogenic switch in which concomitantly chemoattractants such as the aforementioned growth factors, chemokines etc., are produced with VEGF and VCAM-1 being typical examples.

The person skilled in the art is well familiar with methods for monitoring the chemotactic response of e.g. monocytes towards a certain sample containing chemoattractants and/or chemorepellants. Such methods and devices are e.g. described in European patent application EP 07118613.4. Thus, the sample and the monocytes may be placed on a support structure in a distance that is selected such that a chemotactic response of the monocytes can be monitored assuming that the sample will comprise chemorepellants or chemoattractants. The distance can be determined by using appropriate control samples comprising known chemoattractants or repellants. Such a support structure may e.g. be made from glass or plastic materials such as plastics coated with e.g. polylysine. Typically, a microscope cover slide may be a suitable support structure. The movement of the cells towards or away from the sample may then be monitored using different approaches such as visual inspection, internal reflection microscopy (TIRFM).

TIRFM employs the properties of an induced evanescent wave to selectively illuminate a fluorophore in a restricted specimen region immediately adjacent to a glass-fluid, particularly a glass-water or glass-buffer interface. The basis concept of TIRFM requires only an excitation light beam traveling at high incident angle through the support structure on which the cells adhere as has been set out above.

Refractive index differences between the glass and water phases regulate how light is refracted or reflected at the interface as a function of incident angle. At a specific critical angle, the beam of light is totally reflected from the glass/fluid interface, rather than passing through and refracting in accordance with Snell's law. This total reflection generates a very thin electromagnetic field that is usually less than 200 nm in dimension in the fluid medium, which has an identical frequency to that of the incident light.

This field that is also called the evanescent wave or field undergoes exponential intensity decay with increasing distance from the surface. The inventors of the present invention have found that labeling the monocytes for which movement is to be examined with a detectable marker may allow to spectroscopically detecting the evanescent wave generated by TIRFM.

In a preferred embodiment the monocyte cells are thus labeled with fluorescent dyes. To this end one may either label cellular structures such as cell surface receptors with a fluorescent dye or one may use fluorophores that are known to associate with cells. Typical fluorescent markers can include Cy5, Cy3, Texas Red, FITC, Attodye, Cydye, Alexa647, Alexa 488, Alexa 546, Alexa 546, Alexa 594, Alexa 633, GFP, YFP, CFP and dsRED. A particularly suitable fluorophore is the life-cell-tracker from Invitrogen. Other markers may be Q-dots or nanoparticles

If such a labeled monocyte cell is brought into contact with the above-mentioned support structure and the sample, the cells will start to crawl or roll along the surface of the device. If cells which carry at a detectable fluorescent marker and which have adhered to the support structure are analyzed using TIRFM one will observe an increased fluorescence intensity which is amplified as the fluorophore approaches the decaying evanescent field created by TIRFM, stimulating the fluorescent marker. If then the chemotactic stimulus exerts its actions, the cell will start to crawl. This will lead to a reduced interaction between the evanescent field of TIRFM and the fluorescent marker resulting in a reduced detected intensity.

At the same time the leading edge of the cell, which mediates crawling, may get into closer contact and the evanescent field may now then stimulate the fluorophore at a different location of the crawling cell resulting in an increased intensity.

Further, one may use an apparatus as described in EP07118613.4. Such an apparatus comprises:
- a first inlet and a second inlet from which at least first generation of channels originates wherein said first generation comprises at least two first generation channels;
- a first common channel providing communication between each of the at least two first generation channels; and
- at least a second generation of channels comprising at least three second generation channels, each second generation channel having a first end and a second end, the first end of each being in communication with the first common channel and the second end of each being in communication with a second common channel,
- a third inlet; and
- an outlet;
wherein the apparatus is adapted to allow formation of a liquid gradient of a component by connecting the first inlet at least to one fluid comprising a component at a first concentration and by connecting the second inlet at least to one fluid comprising said component at a second concentration wherein the first and second concentration of said component differ; and
wherein said first inlet and said second inlet are adapted to allow for reversal of the orientation of the gradient.

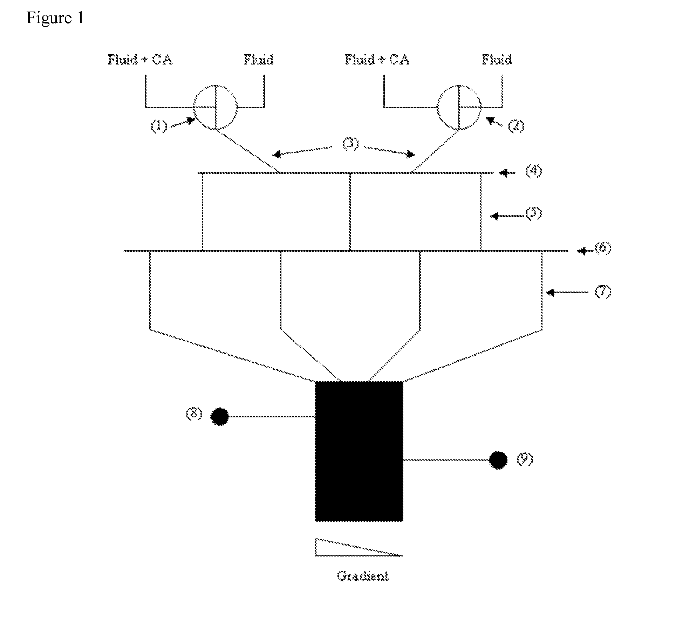

Such a device is depicted in Figure 1.

In a preferred embodiment the first and second inlet will be three way valves with the first and second inlet both being connected to fluids comprising different concentrations of chemotactic agent such as samples taken from a patient at different points in time.

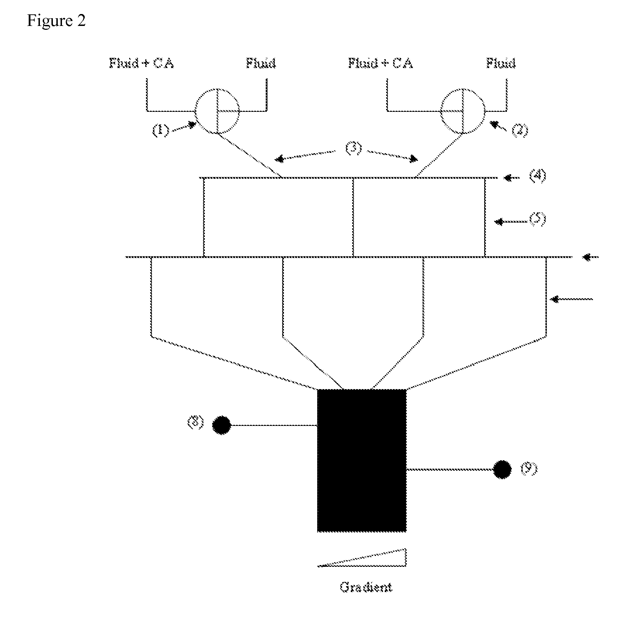

This preferred embodiment of the present invention is depicted in Fig. 2, which describes the same apparatus with different settings of the three-way valve in inlet (1) and inlet (2). In the following the apparatus will be discussed with respect to Fig. 1. Thus, the apparatus comprises a first inlet (1) and a second inlet (2). The first inlet and the second inlet each are connected to fluids comprising a chemotactic agent (CA) at different concentrations. Then a first generation of channels (3) descends from inlet (1) and (2). The ends of this first generation of channels which are opposite to the ends being connected to inlet (1) and (2) connect to a first common channel (4) providing communication between each of the at least two first generation channels. This results in different concentrations of the chemotactic agent along the first common channel.

Then, a second generation of channels (5) comprising at least three-second generation channels descend from the first common channel and are connected to a second common channel (6). Again, along the second common channel (6) the concentration of the chemotactic agent differs. The second common channel may already allow and comprise an inlet for disposing cells into the second common channel and further provide means to monitor movement of said cells. Such means may e.g. a transparent housing, which enables viewing movement of cells by e.g. light microscopy.

In the specific embodiment depicted in Fig. 1, a third generation of channels (7) descends from the second common channel (6) to ultimately establish a gradient in a chamber which has a third inlet (8) that allows of disposing cells in the chamber. In the chamber there is a gradient of different samples and the chamber may comprise means to allow monitoring of the cells' movement along the gradient.

If the three way valves are changed into different positions the direction of the gradient of samples can be reversed (see Fig. 2). The person skilled in the art is aware that these devices may comprise additional generation of channels and common channels being connected by those channels.

As mentioned above once such oscillating gradients are established, one can monitor movement of cells in response to the oscillating gradients of samples. One advantage of monitoring cell movement with respect to oscillating gradient is that one can clearly differentiate between a chemotactic movement, i.e. a response to a chemotactic stimulus versus chemokinetic movement, i.e. the basal random migratory behavior of cells that is independent of a response to a chemotactic stimulus and also designated as "random walk".

There are various parameters by which movement of cells in response to oscillating gradients can be described.

One such parameter is cell trajectories. In order to analyze the trajectories of a cell, the motion of each cell is observed and only the component along the axis of the chemotactic agent gradient which changes direction with the same period as the oscillating gradient is extracted as chemotactic movement.

Cell trajectories for describing chemotactic behavior are a valuable parameter. As has already mentioned above, chemotactic mobility of certain cell types such as monocytes seems to be different for cells that are derived from patients suffering e.g. from prostate tumours of different stages. Thus, by measuring trajectories of monocytes derived from patients at different time points, it is possible to develop profiles that are indicative of the disease state based on the chemotactic migratory behavior of the cells and can thus be used for diagnosis.

The person skilled in the art is aware that other devices may also be used. For example, devices as described in EP07118964.1 may be used. Thus, such an apparatus may be achieved according to the present invention by a device comprising:
- a stimulant gradient chamber for providing a concentration gradient of samples, said stimulant gradient chamber having a cell inlet for the inlet of cells,
- an optical detector for detecting shadows from cells located between a light source and said detector and for generating a detection signal,
- an electrode unit comprising a number of electrode elements surrounding said optical detector,
- a voltage supply unit for supplying the electrode elements of said electrode unit with drive voltages,
- a signal measurement unit for measuring said detection signal, and
- a control unit for controlling said voltage supply unit based on said measured detection signal.

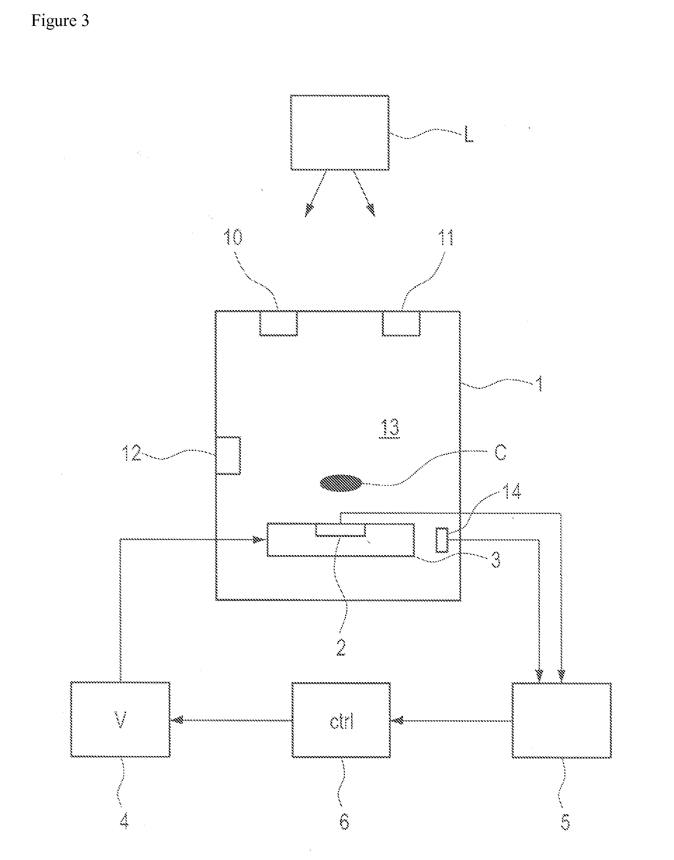

Fig. 3 shows a schematic block diagram of a chemotaxis device according to the present invention including a stimulant gradient chamber 1 for providing a concentration gradient of a stimulant such as serum sample over the areas where the cells C to be monitored are located. To create the concentration gradient, the stimulant gradient chamber 1 generally comprises a sample inlet 10 for the inlet of the stimulant (e.g. a growth factor), a buffer inlet 11 for the inlet of a buffer solution and a cell inlet 12 for the inlet of cells to be monitored into the inner volume 13 of the chamber 1. There are various ways of providing a concentration gradient of a stimulant. These will be explained below in connection with various embodiments of the invention.

An optical detector 2 (or, preferably, an array of optical detectors 2) for detecting shadows from cells located between a light source L (which can be included in the chemotaxis device, can be separately/externally provided or can simply be any available source of ambient light) for providing light and the detector 2 and for generating a detection signal is arranged in the inner volume 13. Further, an electrode unit 3 comprising a number of electrode elements surrounding the optical detector 2 (or, preferably, a plurality of electrode units 3 each comprising a number of electrode elements surrounding one or more of the optical detectors 2 of an array of optical detectors) is arranged in the inner volume 13. Both the optical detector 2 and the electrode unit 3 are only schematically shown in Fig. 3, particular embodiments thereof are shown and explained in more detail below.

For supplying the electrode elements said electrode unit 3 with drive voltages a voltage supply unit 4 is provided. The detection signal generated by the optical detector 2 is measured by a signal measurement unit 5. Based on the measured detection signal the voltage supply unit 4 is controlled by a control unit 6.

To enable a calibration of the available elements of the chemotaxis device, in particular of the signal measurement unit 5 with respect to the available light, a calibration unit 14, e.g. a photodiode, is preferably (but not necessarily) provided in addition.

The following embodiments will be explained referring to an array of optical detectors and a plurality of electrode units enabling the simultaneous monitoring of a plurality of cells. However, as mentioned above, a single optical detector and a single electrode unit is generally sufficient for the monitoring of the movement of cells.

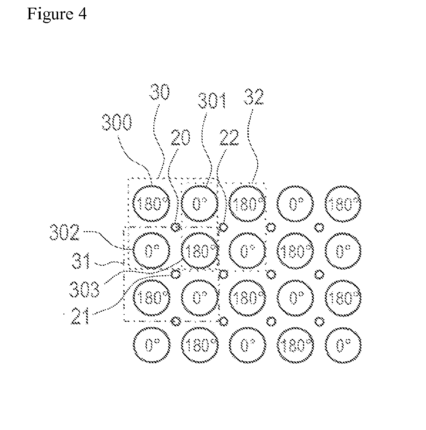

A first embodiment of electrode units including a plurality of quadrupole electrodes 30, 31, 32 is shown in Fig. 4. In this general embodiment an array of optical detectors 20, 21, 22 is shown, each optical detector 20, 21, 22 being surrounded by the electrode elements of a quadrupole electrode 30, 31, 32. For instance, the detector 20 is surrounded by the electrode elements 300, 301, 302, 303 of the quadrupole electrode 30. The quadrupole electrodes are - in this embodiment - overlapping, i.e. the electrodes in the center belong to four quadrupole electrodes which provides a high density.

The electrodes are driven by the supply unit 4 with sine wave voltage profiles with frequencies between 50 kHz and 5 MHz, preferably between 100 kHz and 1 MHz with neighbouring electrode elements (e.g. electrode element 300 as the neighbour of electrode elements 301, 302) having a phase difference of preferably 180°. This well-known method of driving quadrupole electrodes 30, 31, 32 generates an electric field profile that, provided negative dielectrophoresis occurs, results in cells collecting at the central regions of the quadrupole electrodes as indicated by the circles 20, 21, 22 in Fig. 4 where optical detectors are located according to the present invention.

Preferably, the optical detectors are semiconductor elements, e.g. PIN diodes, and are integrated in the substrate, which carries also the quadrupole electrodes, at these locations so that the presence of a cell can be detected. Detection is based on the fact that the shadow from the cells (approximately 12% as determined in experiments) blocks a fraction of illumination light from striking the respective optical detector. This can be detected by monitoring the detection signal of the respective optical detector (e.g. the photo-current in case of a PIN diode).

When a stimulant concentration is present in the chamber 1, the cells being held in the electrode units (e.g. quadrupole units 30, 31, 32) will attempt to migrate in the direction of the gradient. During this attempt at migration the detection signals are monitored with the signal measurement unit 5 (e.g. integrated electronic circuits). If the detection signal indicates a movement of a cell away from the holding position (i.e. if the detection signal shows an increase) then the frequency or voltage amplitude of the drive signal supplied to the relevant quadrupole electrode can be increased in order to prevent movement and/or forcing the cell back to the holding position.

In effect this is a feed-back loop where the dielectrophoretic force from the electrical field is being balanced against the force being exerted by the cell due to chemotaxis. Measurement of the amplitude and/or frequency of the drive voltage signal is a measurement of the mobility of the cell.

The optical detectors are approximately the same size as the cell, and thus a movement of only a few microns is detectable. With a small array of only 100 x 100 sites the mobility of 10000 cells can be measured individually. This allows the distribution of mobility to be measured over a large cell population. As shown above the neighbouring electrode elements can be shared by neighbouring electrode units. This, however, does not allow an independent control. Thus, in an alternative embodiment, the electrode elements belong only to one electrode unit and not to several neighbouring electrode units.

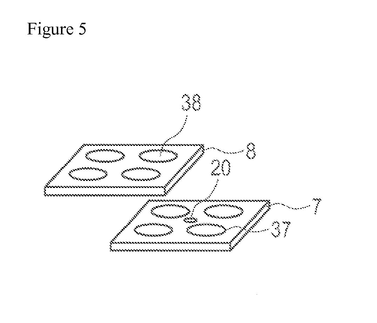

While the embodiment shown in Fig. 4 uses quadrupole electrodes as electrode units, it may be preferable to use octopole electrodes as electrode units as illustrated in Fig. 5. In this embodiment the same array pattern of electrodes 38 are fabricated on a top substrate 8 (on the bottom thereof) arranged in parallel to a bottom substrate 7 carrying the pattern of electrodes 37 on its top surface. As can be seen optical detectors (indicated by one detector 20) are only provided in or on the bottom substrate 7 but not on the top substrate 8. This configuration allows creating a 3D field trap, where the voltage applied on each quadrupole electrode can be adjusted to vary the force with which the cells are "pressed" against the substrate.

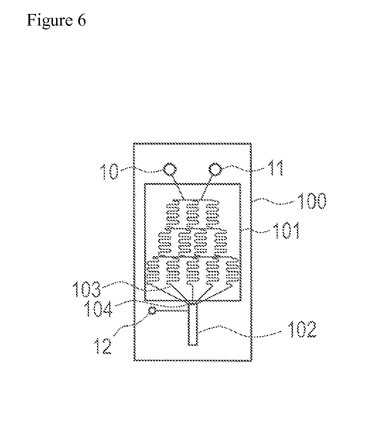

Fig. 6 shows a schematic block diagram of a first embodiment of a stimulant gradient chamber according to the present invention. In this embodiment the stimulant gradient chamber 100 includes a branch structure 101 and a monitoring chamber 102. The branch structure 101 has a plurality of interconnected branches for mixing the stimulant and the buffer solution provided via the stimulant inlet 10 and the buffer inlet 11, respectively, into said concentration gradient of said stimulant. Via a concentration gradient outlet 103 the generated concentration gradient is provided to a concentration gradient inlet 104 of the monitoring chamber 102, into which the cells are loaded via the cell inlet 12. Within said monitoring chamber 102 the array 2 of optical detectors and the plurality of electrode units 3 are arranged.

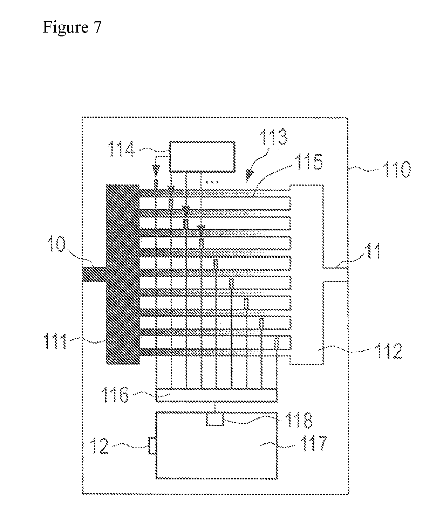

A schematic block diagram of a second embodiment of a stimulant gradient chamber 110 according to the present invention is shown in Fig. 7. In this embodiment the stimulant gradient chamber 110 comprises a stimulant chamber 111 having a stimulant inlet 10 for the inlet of the stimulant, a buffer chamber 112 having a buffer inlet 11 for the inlet of the buffer solution, a plurality of channels 113 connecting said stimulant chamber 111 and said buffer chamber 112, a tapping means 114 for tapping said channels 113 at different tapping locations 115, a concentration gradient outlet 116 for the outlet of the generated concentration gradient, and a monitoring chamber 117 having the cell inlet 12 and a concentration gradient inlet 118 for the inlet of said concentration gradient. Again, within said monitoring chamber 117 the array 2 of optical detectors and the plurality of electrode units 3 are arranged (not shown).

This embodiment is based on the idea to connect two volumes 111, 112 together via many narrow channels 113. By filling one volume 111 with stimulant and the other 112 with buffer a gradient in stimulant concentration is established along the channels 113. By tapping these channels 113 at locations 115 which start near one volume 111 and progressively move towards the other volume 112 it is possible to create a gradient at the outlet 116 where the monitoring chamber 117 is situated.

Other devices that may be used are the well-known Boyden Chamber or the Dunn Chamber.

### Hypothetical experiment 1

In the following hypothetical experiment, the basic concept of the present invention, namely the monitoring of a chemotactic response of monocytes towards the blood serum sample drawn from a patient is explained.

Blood is drawn from a patient (0,1 - 10 ml). This blood may be obtained intravenously or via finger prick into an anticoagulant coated tube. Subsequently the blood is briefly centrifuged, with centrifugation through density gradients being optional to separate the blood cells from the serum. The serum is then removed, aliquoted, frozen in liquid nitrogen and stored at -80°C.

Subsequently, the chemotactic response of monocytes towards such a sample is analyzed in a Boyden Chamber (Wallenberger et al. (2000), Circulation, 102, 185-190).

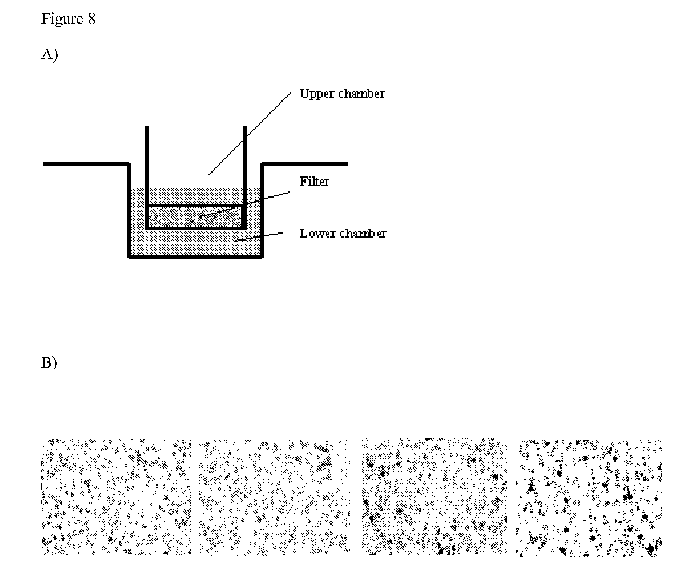

Briefly, the serum is diluted in series into cell media and each sample is assayed for its ability to stimulate monocyte migration. The stimulus, which in the present hypothetical experiment is the serum, is placed in a well of a microwell plate. On this layer, a filter with 5 to 8 µm pores is positioned such that monocytes can migrate through this filter. On the top of the filter paper, one places the cell suspension of monocyte cell lines as U937 or THP-1. The sample is then incubated for 1 to 3 hours at 37°C in an incubator followed by removal of the filter. Subsequently, unbound cells are washed off and the underside of the filter is stained with simple cellular stains such as Wright's or *Geimsa* stain to depict the cells. The cells are then counted. The set up of this Boyden Chamber is depicted in Figure 8A, an expected result, if the serum contains a chemoattractant, is demonstrated in Figure 8B.

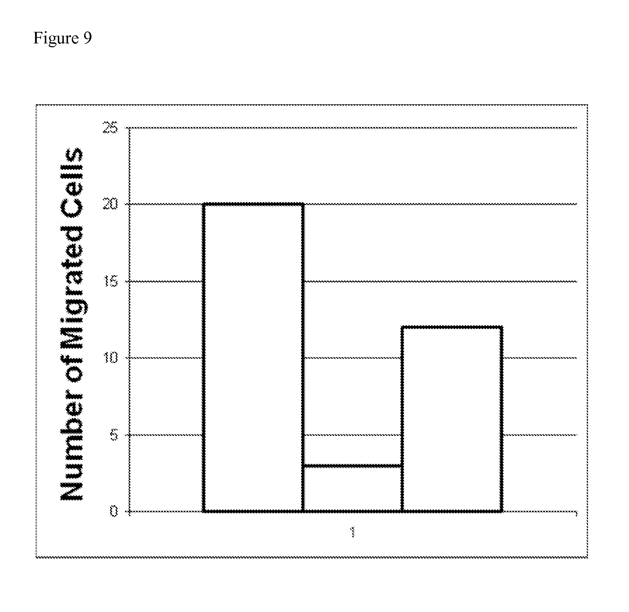

Healthy individuals or patients with only enlarged but non-cancerous prostates would demonstrate a normal monocyte migration response to the serum while patients with early cancers or primary tumors are assumed to demonstrate reduced monocyte migration. Patients however progressing with aggressive angiogenic tumors will demonstrate a subsequent re-increase in monocyte migration. The results are schematically depicted in Figure 9. By monitoring a single patient over time, a sudden increase in the monocyte chemotaxis seems to correlate to prostate cancer progression and indicates a time point for aggressive theoretic intervention.

### Experiment 2

In this experiment, cell lines which are characteristic of different stages of colorectal and prostate cancer were cultured to obtain a conditioned medium This conditioned medium which is used as an approximation of the serum composition of patients suffering from such cancer types was then used to study the migratory response of monocytes.

The following cell lines were utilized and representative of:
1. HT-29: Colorectal adenocarcinoma, primary cancer, grade I.
2. Caco2: Colorectal adenocarcinoma, primary cancer, grade II.
3. Colo205: Colorectal adenocarcinoma, stage IV metastatic cancer.
4. RWPE-1: Non-tumorigenic prostate epithelial cells (represent a benign prostate hyperplasia).
5. LNCaP clone FGC: Metastatic Prostate Carcinoma
6. PC-3: Metastatic stage IV prostate adenocarcinoma
7. THP-1: Monocyte cell line.

All above cell lines are available from the ATCC, and were cultured according to the supplier's recommended protocols. All listed adenocarcinoma and carcinoma cell lines, with the exception of RWPE-1, produce tumors consistent with the above said stage of cancer, when injected into mice.

### Conditioned Medium Preparation

All colorectal and prostate cancer cell lines were grown to 70-80% confluence under 5% CO₂ at 37 degrees C, in ATCC recommended mediums (RPMI medium for THP-1, HT29, CaCo2, Colo205, and LNCaP clone FGC, Ham's F12 medium for PC-3 cells, and Keratinocyte Serum Free Medium for RWPE-1 cells, all containing 10% fetal calf serum).. The medium was then changed and fresh medium was placed on the cells for a period of 24 hours. The following day, this medium was harvested, centrifuged at 300 X g for 5 min and sterile filtered through a 0.2 um pore filter. Medium was stored in 5 mL aliquots at -80 degrees C until used. This conditioned medium is representative of the plasma of patients suffering from the various stages of colorectal and prostate cancer described above. Each tumor type and stage produces a unique cocktail of chemokines and cytokines that the tumor excretes in to the surrounding tissues and blood, where it can interact with the patient's monocytes and other circulating cells.

### Migration Assay

To mimick the effects of the cancer patient serum on monocyte migration, healthy logarithmically growing THP-1 monocytes (ATCC) were utilized. 2 mLof THP-1 cells were seeded in 10 wells of 2X 6 well plates at a concentration of 5 X 10e5 cells/mL. In 2 of the wells, 2 mL of normal THP-1 medium (RPMI with 10% fetal calf serum) was added. This is known as the "non-treated control". To one well, 2 mL of HT-29 conditioned medium was added. To one well, 2 mL of Caco2 conditioned medium was added. To one well, 2 mL of Colo205 conditioned medium was added. To one well, 2 mL of RWPE-1 conditioned medium was added. To one well, 2 mL of LNcaP clone FGC conditioned medium was added. To one well, 2 mL of PC-3 conditioned medium was added. The 6 well plates were then incubated over night in a 5% CO₂ atmosphere at 37° C.

The next day, the THP-1 monocytes from each well were centrifuged for 5 minutes at 300 X g, the supernatant was discarded and the monocytes were resuspended in 1 mL fresh RPMI with 10% fetal calf serum medium, each. Cells were counted to ensure that equal cell concentrations of each cell treatment were present. These 1 mL cell suspensions were adjusted to contain 1 X 10⁶ cells/mL.

Each sample was then examined for migration potential of the monocytes towards the strong monocyte chemoattractant, MCP-1 (abcam). Modified 8 µm pore, 96-well boyden chambers were purchased from NeuroProbe and used according to the manufacturers recommended protocol.

For each sample (treated monocytes from the various stages of colorectal and prostate cancer) the bottom well of the boyden chamber contained 30 uL of fresh RPMI medium with 10% fetal calf serum and 20 ng/mL MCP-1. On the top of the chamber 25 uL of the various treated THP-1 monocyte suspensions (at 1 X 10e6 cell/mL) was added. The top and bottom chamber is separated by a membrane containing 8 um pores through-which the monocytes can migrate towards the MCP-1 signal. The boyden chambers were incubated at 37 degrees C under 5% CO₂ conditions for 2 hours. Following this incubation, the cells were scrapped from the top of the chamber with a cell scraper, and the 96-well boyden chamber was centrifudged for 10 min at 400 X g. This ensured that only cells that migrated through the filter would be found in the bottom chambers of the 96-well plate. To each sample, 1 µM of Cell Tracker Green stain was added (invitrogen). Cell tracker green is a living cell stain that produces a strong green fluorescence when internalized by living cells only.

The fluorescence was then measured for each well of the 96-well boyden chamber using a standard well-plate reader (note: only 10 wells were utilized since only 10 samples were examined). Since the fluorescence signal is only produced by living cells, the overall fluorescence intensity per well is directly proportional to the number of cells that have migrated through the 8 um pore membrane.

### Results

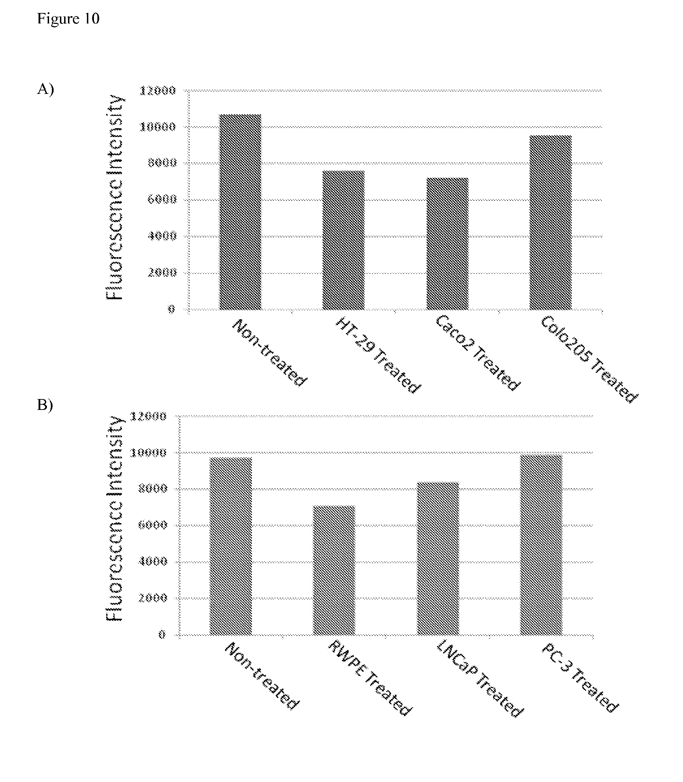

The detected fluorescence intensities are shown in Figure 10. Again, fluorescence intensity is directly proportional to the number of migrated monocytes.

As can be seen in Figure 10, the primary or benign forms of cancer (HT-29, Caco2 and RWPE-1) produce a chemo-repulsion effect on the monocytes, hence the level of THP-1 migration is lower than that of healthy non-treated cells. For the malignant forms of the tumors, i.e. the metastatic tumors, one observes an increase or re-induction in the migration of the monocytes.

## Claims

1. Method of diagnosing and/or monitoring development of an adenocarcinoma-related cancer comprising at least the steps of:
a. Providing at least a sample of at least one individual suspected of suffering from or of developing an adenocarcinoma-related cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from at least one healthy individual;
e. Comparing the chemotactic responses of said blood cells;
f. Deciding on the occurrence and/or state of an adenocarcinoma-related cancer on the basis of the results obtained in e.

2. Method of data acquisition comprising at least the steps of:
a. Providing at least a sample of at least one individual suspected of suffering from or of developing an adenocarcinoma-related cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from at least one healthy individual;
e. Comparing the chemotactic responses of said blood cells.

3. Method of diagnosing and/or monitoring development ofan adenocarcinoma-related cancer comprising at least the steps of:
a. Providing at least a sample of at least one individual suspected of suffering from or of developing an adenocarcinoma-related cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from the same said at least one individual at later point in time;
e. Comparing the chemotactic responses of said blood cells;
f. Deciding on the state of an adenocarcinoma-related cancer on the basis of the results obtained in e.

4. Method of data acquisition comprising at least the steps of:
a. Providing at least a sample of at least one individual suspected of suffering from or of developing an adenocarcinoma-related cancer;
b. Bringing said sample in contact with blood cells such that a chemotactic response of said blood cells is inducible by said sample;
c. Monitoring the chemotactic response of said blood cells with respect to said sample;
d. Repeating steps a. to c. with a sample obtained from the same said at least one individual at later point in time;
e. Comparing the chemotactic responses of said blood cells.

5. Method according to claims 1 or 3 wherein the sample is not in direct contact with the human body.

6. Method according to any of claims 1 to 4 wherein said adenocarcinoma-related cancer is a breast cancer, colon and rectal cancer, prostate cancer, lung cancer, kidney cancer, stomach cancers, pancreas cancer, cervix cancer, vaginal cancer,head and neck cancers or urachus cancer.

7. Method according to any of claims 1 to 4 wherein the sample is derived from human blood.

8. Method according to any of claims 1 to 4 wherein the blood cells are selected from monocytes, T-lymphocytes, stem cells, progenitor cells and neutrophils.

9. Method according to claim 8 wherein the blood cells are cell culture lines of monocytes.

10. Method according to any of claims 1 to 4 wherein the chemotactic behavior is determined by measuring monocytes moving towards and/or away from said sample.

11. Method according to claim 3 wherein the results obtained in e. are used for deciding on the benignity or malignancy of an adenocarcinoma-relatedcancer tumor.
